Europäisches Patentamt

European Patent Office

Office européen des brevets

(19)

(11) EP 0 796 617 A1

(12) # DEMANDE DE BREVET EUROPEEN

(43) Date de publication:
24.09.1997 Bulletin 1997/39

(51) Int Cl.6: A61K 31/34

(21) Numéro de dépôt: 97400593.6

(22) Date de dépôt: 17.03.1997

(84) Etats contractants désignés:
AT BE CH DE DK ES FI FR GB GR IE IT LI LU MC
NL PT SE
Etats d'extension désignés:
AL LT LV RO SI

(30) Priorité: 18.03.1996 FR 9603357

(71) Demandeur: SANOFI
75008 Paris (FR)

(72) Inventeurs:
• Frangin, Gérald
34090 Montpellier (FR)
• Munoz, Alain
21121 Fontaine-Les Dijons (FR)

(74) Mandataire: Le Guen, Gérard et al
CABINET LAVOIX
2, place d'Estienne d'Orves
75441 Paris Cédex 09 (FR)

(54) **Utilisation de composés antiarythmiques dansla réduction de la mortalité post infarctus**

(57)     L'invention a pour objet l'utilisation de dérivés de benzofuranne à activité antiarythmique en particulier l'amiodarone ou la dronédarone, ou d'un sel pharmaceutiquement acceptable de ces dérivés, pour la préparation de compositions pharmaceutiques destinées à réduire la mortalité d'origine cardiaque en particulier la mortalité cardiaque d'origine arythmique et la mort subite chez des patients présentant une fonction ventriculaire gauche réduite après infarctus du myocarde, sans troubles du rythme nécessitant un traitement antiarythmique.

## Description

La présente invention concerne, d'une manière générale, l'utilisation de composés antiarythmiques, pour la prévention de la mortalité après infarctus du myocarde.

En particulier, l'invention a pour objet l'utilisation de dérivés du benzofuranne connus pour leur activité antiarythmique, en particulier l'amiodarone, ou de sels pharmaceutiquement acceptables de ces dérivés, pour la prévention de certains types de mortalité après infarctus récent du myocarde, notamment la mortalité d'origine cardiaque en particulier la mortalité cardiaque d'origine arythmique.

La mortalité après infarctus du myocarde apparaît la plus élevée, de l'ordre de 15%, dans les 6 à 12 premiers mois après l'accident aigu tandis qu'elle diminue par la suite pour s'aligner sur la mortalité enregistrée dans la maladie coronarienne stable et chronique.

Dans ce contexte, l'étendue de la masse myocardique détruite, pouvant se traduire par la fraction d'éjection, ainsi que les arythmies ventriculaires sont reconnues comme des facteurs de risque majeurs.

Par conséquent, une thérapie médicale destinée à prévenir le décès après infarctus du myocarde peut s'avérer particulièrement indiquée durant la première année après la survenue de cet accident cardiaque bien qu'il y ait discussion possible sur la durée même de cette thérapie prophylactique.

De nombreuses études réalisées chez des patients ayant présenté un infarctus du myocarde attestent toutefois que le dysfonctionnement ventriculaire gauche, les arythmies ventriculaires et l'importance de la maladie coronarienne concourent de manière indépendante au risque de mortalité globale et de mort subite, le dysfonctionnement ventriculaire gauche étant le facteur de risque essentiel (Multicenter Postinfarction Research Group -N. Engl. J. Med. 1983; 309: 331-336).

Ainsi, en dépit des progrès importants réalisés ces dernières années dans le traitement de l'infarctus myocardique, le pronostic reste assez réservé chez les malades qui présentent une altération majeure de la fonction ventriculaire se reflétant par un abaissement marqué de la fraction d'éjection à ce niveau.

Bien que ce pronostic soit plus favorable chez les patients avec fraction d'éjection supérieure à 40%, il se détériore progressivement à mesure que ce paramètre tend vers le seuil critique de 40%. La mort, souvent subite chez ces patients, est très probablement causée dans la plupart des cas, par une fibrillation ventriculaire dégénérant en tachycardie ventriculaire léthale.

Dans ces cas, il semble, par conséquent, logique d'essayer de prévenir ces troubles ventriculaires par l'administration d'agents antiarythmiques. Malheureusement, toutes les tentatives pour diminuer la mortalité post infarctus avec les agents antiarythmiques de classe I (classification de Vaughan-Williams) les plus usités ont échoué notamment chez des patients porteurs d'arythmies ou avec fonction ventriculaire gauche abaissée (The CAST Investigators - N. Engl. J. Med. 1989; 321 : 406-412).

Au contraire, ces agents antiarythmiques devraient être contre-indiqués puisqu'au lieu de diminuer la mortalité après infarctus du myocarde ils accroissent en fait le nombre de morts subites, probablement en raison de leurs effets arythmogènes.

A la suite de ces observations, la grande majorité des cardiologues se sont montrés sceptiques et même opposés au concept de prévention de la mort subite après infarctus du myocarde par l'administration d'agents antiarythmiques.

L'amiodarone, un puissant correcteur des troubles du rythme, largement utilisé dans cette indication, est capable de supprimer de manière importante les contractions ventriculaires prématurées complexes et la tachycardie ventriculaire soutenue tout en ne présentant qu'un effet inotrope négatif peu marqué et en étant nettement moins arythmogène que les antiarythmiques de classe I.

Toutefois, l'amiodarone se différentie des autres agents antiarythmiques par un mécanisme d'action différent : sa faculté de prolonger la durée du potentiel d'action et d'allonger la période réfractaire de la cellule myocardique le situe dans la catégorie des antiarythmiques de classe III de Vaughan-Williams.

S'il est parfaitement reconnu que l'amiodarone est un agent bénéfique dans les arythmies potentiellement mortelles souvent réfractaires aux autres antiarythmiques, son efficacité a également été évaluée dans la prévention des décès survenant après infarctus du myocarde.

Par exemple, L. Ceremuzinski et coll. (J. Am. Coll. Cardiol. 1992; 20 : 1056-1062) ont enregistré, après administration d'amiodarone à la dose de 200mg / jour pendant un an, une réduction significative de la mortalité post infarctus et des arythmies ventriculaires chez des patients à haut risque présentant une contre-indication à la prescription de β-bloquants tandis que dans l'étude CAMIAT (Circulation 1991; 84 : 550-557), J.A. Cairns et coll. ont constaté, à la dose de 300 à 400mg / jour, une tendance de l'amiodarone à présenter un effet bénéfique vis-à-vis notamment de la mortalité totale ou la mortalité d'origine cardiaque chez des patients présentant des extrasystoles ventriculaires après infarctus du myocarde.

Dans l'étude BASIS (J. Am. Coll. Cardiol. 1990; 16 : 1711-1718), F. Burkart et coll., quant à eux, ont étudié pendant une année les effets de l'amiodarone à la dose de 200mg / jour chez des survivants d'infarctus du myocarde porteurs d'arythmies. Toutefois, les effets bénéfiques enregistrés doivent être pondérés par un manque d'efficacité observé chez les patients avec fonction ventriculaire gauche diminuée c'est-à-dire avec fraction d'éjection inférieure ou égale à 40%.

L'intérêt essentiel de cette étude réside, par conséquent, dans la mise en évidence d'un effet de l'amiodarone sur la survie de patients porteurs d'arythmies ventriculaires complexes asymptomatiques avec fonction ventriculaire gauche préservée (fraction d'ejection ≥ 40%).

En conséquence, la fraction d'éjection du ventricule gauche apparaît davantage engagée dans le pronostic de survie du patient que les arythmies ventriculaires en accord avec C.S. Chakko et coll. (Am. Heart J. 1985; 109 : 497-504).

D'autre part des essais cliniques menés chez des patients n'ayant pas montré d'infarctus du myocarde ont mis en lumière des résultats parfois divergents.

Par exemple, de petites études préliminaires entreprises avec l'amiodarone chez des patients souffrant de cardiomyopathies, d'arythmies ventriculaires ou d'insuffisance cardiaque ont fourni des résultats contradictoires : Neri et coll. (Am. Heart J. 1987; 113 : 707-715) ont enregistré une survie améliorée, Cleland et coll. (Br. Heart J. 1987; 58 : 572-582) ont rapporté une incidence diminuée de la mort subite tandis que Nicklas et coll. (Am. Heart J. 1991; 122 : 1016-1021) ainsi que Stewart et coll. (Brit. Heart J. 1989; 61 : 459-460) n'ont constaté aucune amélioration.

De même, dans une autre étude pratiquée chez des patients atteints d'insuffisance cardiaque sévère en l'occurrence, l'étude GESICA, H.C. Doval et coll. (Lancet 1994 ; 344 : 493-498) ont montré que l'amiodarone, à la dose de 300mg / jour, pendant 2 ans a permis d'obtenir, une réduction significative de la mortalité dans le groupe traité par rapport au groupe contrôle qu'il s'agisse de la mort subite ou de la mortalité due à l'insuffisance cardiaque progressive.

Enfin, S.N. Singh et coll. ont publié dans N. Engl. J. Med. 1995; 333 : 77-82, les résultats de l'étude clinique CHF-STAT s'étendant sur 45 mois avec des doses journalières de 300mg d'amiodarone chez des patients porteurs d'arythmies ventriculaires asymptomatiques et souffrant d'insuffisance cardiaque modérée d'origine ischémique ou non ischémique.

Bien que ce composé se soit révélé efficace pour supprimer les arythmies ventriculaires et améliorer la fonction ventriculaire, cette étude a montré, d'une manière tout à fait inattendue, que l'amiodarone n'a aucun effet, après 2 ans, ni sur la mort subite, ni sur la prolongation de la survie de patients montrant une insuffisance cardiaque modérée.

Toutefois, une tendance à réduire la mortalité chez des patients avec cardiomyopathie non ischémique a pu être relevée (fraction d'éjection ≥ 40%).

Des tentatives d'interprétation des résultats apparemment contradictoires mis en lumière dans ces deux derniers essais ont été formulées par G.Breithardt (N. Engl. J. Med. 1995; 333 : 121-122) et par S.C. Hammill et coll. (Heart 1996 ; 75 : 6-7).

Ces auteurs ont fait remarquer que la différence la plus significative entre les deux études repose sur la nature même de la maladie cardiaque sous-jacente.

En fait, la grande majorité des patients dans l'essai CHF-STAT présentaient une cardiomyopathie ischémique alors, qu'à l'inverse, une majorité de patients dans l'étude GESICA étaient atteints de cardiomyopathie non ischémique.

En conséquence, et pour cette raison, les malades de l'étude CHF-STAT n'ont probablement pas tiré avantage de l'amiodarone. La tendance remarquée dans l'étude BASIS citée précédemment où un manque d'efficacité de ce composé avait été observé chez des patients avec fraction d'éjection < 40% se trouverait donc confirmée par ces essais.

On peut donc conclure, à la suite de ces données cliniques, que la cause de l'insuffisance cardiaque sous-jacente peut jouer un rôle prédominant dans la détermination d'un effet bénéfique de l'amiodarone sur la prophylaxie de la mortalité.

On mentionnera par ailleurs le brevet FR 2 626 176 qui concerne des compositions destinées au traitement de l'insuffisance coronaire par association d'amiodarone et d'au moins un vasodilatateur et un β-bloquant.

Selon ce brevet, l'amiodarone et l'isosorbide dinitrate, pris isolément, ne montrent pas, par rapport à un groupe contrôle, une diminution de la mortalité ni à 7 jours ni à 1 an alors que l'administration précoce, en début d'infarctus, de l'association isosorbide dinitrate / amiodarone suivie d'une administration prolongée de cette même association réduirait la mortalité à 1 an dans l'infarctus du myocarde.

Néanmoins aucun résultat comparatif n'y est rapporté ce qui permettrait de soutenir cette conclusion.

Cette affirmation apparaît par conséquent comme purement spéculative et d'autant plus douteuse qu'elle ne figure plus dans la demande de brevet WO 90/09176 du même inventeur qui décrit, postérieurement, les mêmes compositions destinées au traitement de l'insuffisance coronaire.

Enfin, on signalera la demande de brevet WO 95/09625 se rapportant à l'utilisation de l'amiodarone pour le traitement de patients souffrant d'insuffisance cardiaque qu'ils soient porteurs ou non d'arythmies ventriculaires. L'amiodarone y est éventuellement associée à d'autres agents cardioactifs.

Cependant, on ne fait aucune référence, dans cette demande de brevet, à un dysfonctionnement ventriculaire gauche comme facteur essentiel de cause de mortalité de sorte qu'il est totalement impossible de déduire ou encore de prévoir le comportement de l'amiodarone, associée ou non à d'autres agents cardioactifs, en présence de ce seul facteur de risque se traduisant par une fonction ventriculaire gauche amoindrie.

A partir des résultats regroupés ci-dessus, on peut affirmer que, dans une certaine mesure, un bénéfice de l'amiodarone a pu être démontré sur la prévention de la mortalité après infarctus du myocarde ou non et ce, chez des malades présentant des troubles du rythme et/ou certaines formes d'insuffisance cardiaque.

Toutefois, on n'a jamais rapporté ni même publié jusqu'à présent un avantage quelconque de l'amiodarone dans la prophylaxie des décès par administration précoce de cet agent après la phase aigüe de l'infarctus du myocarde notamment dans la prophylaxie de la mortalité d'origine cardiaque, en particulier la mortalité cardiaque d'origine arythmique, en tenant compte, comme seul facteur de risque prédominant et mortel, d'un dysfonctionnement ventriculaire gauche se traduisant par une fraction d'éjection basse.

On signalera cependant les publications de A. Munoz (Arch. Mal. Coeur 1991; 94 (II) : 67-69) et de J.A. Camm et coll. (Am. J. Cardiol. 1993; 72 : 95F-98F) qui décrivent un protocole d'étude clinique impliquant l'évaluation de l'amiodarone comme agent de prévention de la mortalité après infarctus du myocarde chez des patients ne présentant pas d'arythmies lors de l'incorporation dans l'essai mais une fonction ventriculaire gauche altérée.

L'étude en question nécessitant plusieurs années, aucun résultat quel qu'il soit n'a encore été publié qui permettrait d'affirmer ou d'infirmer les hypothèses qui lui servent de fondement.

Aussi, peut-on conclure de l'ensemble des études rapportées précédemment, que le comportement de l'amiodarone vis-à-vis de la mortalité de patients à risques, qu'ils soient ou non dans une période post infarctus, est loin d'être élucidé d'autant que des résultats parfois surprenants et divergents ont été enregistrés qui ne permettent la formulation que de simples suppositions sur les aspects encore méconnus de cet antiarythmique.

Or, on a maintenant trouvé que l'amiodarone et ses sels pharmaceutiquement acceptables sont capables de prévenir de manière significative et avec moins d'effets arythmogènes que les antiarythmiques de classe I, certains types de mortalité notamment la mortalité d'origine cardiaque en particulier la mortalité cardiaque d'origine arythmique chez des patients présentant après infarctus du myocarde, une fonction ventriculaire gauche diminuée, c'est-à-dire des patients avec cardiomyopathie ischémique, sans troubles du rythme symptomatiques.

Par conséquent, on peut affirmer avoir mis en évidence pour la première fois, dans le cadre de la présente invention, la possibilité de prévenir la mortalité cardiaque d'origine arythmique notamment la mort subite dans la période post infarctus, au moyen d'un agent antiarythmique, en l'occurrence l'amiodarone ou ses sels pharmaceutiquement acceptables, chez des patients avec facteur de risque majeur mais sans arythmies symptomatiques.

Cette prévention de la mortalité cardiaque d'origine arythmique ainsi relevée se traduit concrètement par une réduction du taux de mortalité d'origine arythmique, en particulier une réduction du taux de morts subites.

Ainsi, l'invention se rapporte à l'utilisation, comme principe actif, d'un dérivé de benzofuranne à activité antiarythmique en particulier l'amiodarone ou la dronédarone ou d'un sel pharmaceutiquement acceptable de ce dérivé, de préférence le chlorhydrate, pour la préparation d'une composition pharmaceutique destinée à réduire la mortalité d'origine cardiaque chez des patients présentant une fonction ventriculaire gauche réduite après infarctus du myocarde, sans troubles du rythme nécessitant un traitement antiarythmique.

Par "dérivé de benzofuranne à activité antiarythmique", on désigne, dans le cadre de la présente invention, un composé benzofurannique choisi parmi ceux décrits dans les brevets US 3248401, US 5223510 et EP 338746 ainsi que dans les demandes de brevet WO 88/07996, WO 89/02892, WO 90/02743 et WO 94/29289.

De l'ensemble de ces composés, on peut citer par exemple le 2-n-butyl-3-[4-(3-di-n-butylaminopropoxy)benzoyl]-5-méthylsulfonamido-benzofuranne ou dronédarone et ses sels pharmaceutiquement acceptables décrits dans le brevet US 5223510.

Cependant, on préfère généralement, comme dérivé de benzofuranne à activité antiarythmique, l'amiodarone et ses sels pharmaceutiquement acceptables décrits dans le brevet US 3248401.

On entend d'ailleurs par "amiodarone", dans le contexte de la présente invention, le 2-n-butyl-3-(4-diéthylaminoéthoxy-benzoyl)benzofuranne sous forme basique ou sous forme de complexes d'inclusion dans des cyclodextrines, leurs éthers ou leurs esters.

L'utilisation des métabolites actifs des dérivés de benzofuranne à activité antiarythmique dont il est question précédemment, fait également partie de la présente invention. Comme exemple de tels métabolites on peut citer le 2-n-butyl-3-(4-éthylaminoéthoxy-benzoyl)benzofuranne ou N-deséthylamiodarone et ses sels pharmaceutiquement acceptables tels que décrits dans le brevet FR 2550091 et le 2-n-butyl-3-[4-(3-n-butylaminopropoxy)benzoyl]-5-méthylsulfonamido-benzofuranne ou N-desbutyldronédarone et ses sels pharmaceutiquement acceptables décrits dans le brevet US 5223510.

De même, l'appellation "mortalité d'origine cardiaque" recouvre, dans le cadre de l'invention, la mortalité due à toutes causes cardiaques (tous décès exceptés ceux dus à une cause non cardiaque) notamment, et de manière plus particulière, la mortalité cardiaque d'origine arythmique.

Par "mortalité cardiaque d'origine arythmique", on désigne d'ailleurs la mortalité subite ou non due à toute cause arythmique c'est-à-dire soit le décès se produisant dans l'heure ou moins d'une heure après l'apparition de nouveaux symptômes en l'absence d'insuffisance ventriculaire gauche agravée ou de choc soit le décès sans témoin d'un patient apparemment stable, soit la fibrillation ventriculaire fatale ("non ressuscitée").

L'appellation "mortalité cardiaque d'origine arythmique", recouvre également, dans le cadre de l'invention, les arrêts cardiaques non fatals ("ressuscités" après défibrillation), c'est-à-dire les événements arythmiques non fatals causant un arrêt cardiaque.

De plus, l'expression "mortalité cardiaque d'origine arythmique" inclut également de manière plus spécifique, la "mortalité cardiaque subite d'origine arythmique" ou "mortalité subite d'origine arythmique" ou "mort subite", à savoir le décès se produisant dans l'heure ou moins d'une heure après l'apparition de nouveaux symptômes en l'absence d'insuffisance ventriculaire gauche aggravée ou le décès fortuit d'un patient sans symptômes ou avec symptômes cardiovasculaires stables survenu sans témoin et dans les 24 heures de la connaissance de cet événement.

On précisera également que l'expression «patient présentant une fonction ventriculaire gauche réduite après infarctus du myocarde, sans troubles du rythme nécessitant un traitement antiarythmique» a pour signification un patient, qui doté d'une fonction ventriculaire gauche diminuée après infarctus du myocarde, peut éventuellement présenter des troubles du rythme asymptomatiques c'est-à-dire sans signification pathologique mais pas de troubles du rythme relevant d'un traitement antiarythmique.

Enfin par "fonction ventriculaire gauche réduite", on entend essentiellement une fonction ventriculaire gauche déficiente se reflétant par une fraction d'éjection $\leq$ 40%.

Comme il sera décrit plus en détails par la suite, on a relevé au cours d'un essai impliquant plusieurs centaines de patients, que l'amiodarone de préférence sous forme de son chlorhydrate, administrée journellement après la phase aiguë de l'infarctus du myocarde ne provoque pas d'effet délétère sur une période de 24 mois.

En outre, on a trouvé que cet agent antiarythmique tend à prévenir la mortalité d'origine cardiaque dans certains cas spécifiques, par exemple chez des patients avec fraction d'éjection supérieure à 30% et inférieure ou égale à 40% dans la période post infarctus inaugural c'est-à-dire chez des patients n'ayant pas fait plusieurs infarctus (sans histoire d'infarctus).

De même, on a pu mettre en évidence que le chlorhydrate d'amiodarone peut prévenir, de manière significative, sur une période d'au moins 2 ans, la mortalité cardiaque d'origine arythmique, subite ou non, comprenant ou pas les arrêts cardiaques non fatals, puisqu'on a pu enregistrer une réduction de 28 à 30% de ce type de mortalité à 2 ans.

Par ailleurs, on a trouvé que le chlorhydrate d'amiodarone peut réduire également de façon tout à fait significative, la mortalité subite d'origine arythmique ou mort subite sur une période de 24 mois (37% de réduction à 24 mois).

Cet effet prophylactique qui a été observé aussi bien chez des patients avec fraction d'éjection supérieure à 30% et inférieure ou égale à 40% s'est même révélé encore plus marqué chez des patients avec fraction d'éjection ventriculaire gauche fortement diminué c'est-à-dire < 30 %.

Pour leur utilisation en thérapeutique, les dérivés de benzofuranne à activité antiarythmique selon l'invention sont généralement introduits dans des compositions pharmaceutiques.

Ces compositions pharmaceutiques peuvent être présentées sous toute forme convenant à leur administration en thérapie humaine notamment pour administration par voie orale, sublinguale, nasale, inhalée, parentérale, topique, transdermique ou rectale.

Généralement, on utilise la voie orale ou parentérale mais, de préférence la voie orale. Pour ce qui concerne l'unité d'administration celle-ci peut prendre la forme, par exemple d'un comprimé sécable ou non, d'une dragée, d'une capsule, d'une gélule, d'une poudre, d'une suspension ou d'un sirop pour l'administration orale, d'un suppositoire pour l'administration rectale, d'une solution ou suspension pour l'administration parentérale ou d'un dispositif appelé communément "patch" pour l'administration transdermique.

Les compositions pharmaceutiques selon l'invention pourront avantageusement comprendre, par unité d'administration, de 50 à 600mg de principe actif notamment de 150 à 250mg pour l'administration orale, de 10 à 250mg de principe actif pour l'administration parentérale ou encore de 50 à 200mg de principe actif pour l'administration rectale.

On peut constituer notamment des compositions pharmaceutiques contenant l'amiodarone, la dronédarone ou un de leurs sels pharmaceutiquement acceptables à raison de 50 à 500mg pour l'administration orale plus particulièrement des compositions orales renfermant de 150 à 250mg d'amiodarone ou d'un de ses sels pharmaceutiquement acceptables ou encore de 50 à 150mg de dronédarone ou d'un de ses sels pharmaceutiquement acceptables.

Selon la voie d'administration choisie, les compositions pharmaceutiques en question seront préparées, de manière conventionnelle en associant le principe actif tel que l'amiodarone ou la dronédarone ou un de leurs sels pharmaceutiquement acceptables, avec un excipient ou véhicule pharmaceutique approprié, celui-ci pouvant être constitué, par exemple d'au moins un ingrédient sélectionné parmi les substances suivantes:

lactose, amidons, talc, stéarate de magnésium, polyvinylpyrrolidone, acide alginique, silice colloïdale, polysorbate, mannitol, dihydrogénophosphate sodique, eau distillée, alcool benzylique ou agents édulcorants.

Pour la préparation d'une composition pharmaceutique orale, on peut par exemple mélanger le principe actif avec les excipients ou véhicules pharmaceutiques sélectionnés puis procéder à une granulation à l'état sec ou humide.

Selon la nature de l'excipient utilisé, on peut si nécessaire, obtenir par simple mélange une poudre susceptible d'être convertie directement en comprimés. On peut introduire cette poudre ou ce granulé directement dans des capsules ou gélules ou, de préférence, les transformer par compression en noyaux de comprimés.

A titre d'exemple, on peut préparer des compositions pharmaceutiques dosées à 200mg de chlorhydrate d'amiodarone pour une administration par voie orale par exemple sous la forme de comprimés, sécables ou non, par granulation et compression des ingrédients suivants :

_Exemple 1_

| Chlorhydrate d'amiodarone | 200mg |
|---|---|
| Lactose | 96mg |
| Amidon de maïs | 60mg |
| Polyvinylpyrrolidone | 12mg |
| Silice colloïdale anhydre | 2,4mg |
| Stéarate de magnésium | 4,6mg |
| | 375mg |

_Exemple 2_

| Chlorhydrate d'amiodarone | 200mg |
|---|---|
| Lactose | 71mg |
| Amidon de maïs | 66mg |
| Polyvinylpyrrolidone | 6mg |
| Silice colloïdale anhydre | 2,4mg |
| Stéarate de magnésium | 4,6mg |
| | 350mg |

De même, on peut constituer une composition pharmaceutique contenant 106mg de chlorhydrate de dronédarone pour une administration orale notamment sous forme d'une gélule :

*Exemple 3*

| Chlorhydrate de dronédarone | 106mg |
|---|---|
| (soit 100mg de dronédarone base) | |
| Amidon de maïs | 138,5mg |
| Talc | 50mg |
| Silice colloïdale anhydre | 1,25mg |
| Stéarate de magnésium | 2,5mg |
| Lactose | 201,75mg |
| | 500mg |

Pour une administration parentérale, on peut préparer également des compositions pharmaceutiques dosées à 150mg de chlorhydrate d'amiodarone ou 10,6mg de chlorhydrate de dronédarone, par exemple sous la forme d'une solution injectable de formulation :

*Exemple 4*

| Chlorhydrate d'amiodarone | 150mg |
|---|---|
| Polysorbate 80 | 300mg |
| Alcool benzylique | 60mg |
| Eau pour préparation injectable Q.S.* | 3ml |

\* Q.S.: quantité suffisante pour

*Exemple 5*

| Chlorhydrate de dronédarone (soit 10mg de dronédarone base) | 10,6mg |
|---|---|
| Mannitol | 200mg |
| Dihydrogénophosphate sodique anhydre | 120mg |
| Eau pour préparation injectable Q.S.* | 10ml |

\* Q.S.: quantité suffisante pour

Lors de la recherche d'un effet prophylactique sur la mortalité post infarctus, en accord avec l'invention, le principe actif, plus spécialement l'amiodarone ou ses sels pharmaceutiquement acceptables, peut être administré à des doses journalières variant de 100 à 800mg par voie orale, de préférence de 200 à 400mg.

Dans le cas particulier de l'administration par voie orale de l'amiodarone, ou de ses sels pharmaceutiquement acceptables, des doses journalières supérieures à 400mg par exemple des doses de 400 à 800mg notamment des doses de 600 à 800mg, sont réservées aux traitements de courte durée par exemple aux traitements d'attaque nécessitant une dose de charge importante en principe actif alors que les doses journalières inférieures ou égales à 400mg, par exemple des doses de 200 à 300mg de préférence des doses de 200mg représentent davantage des doses d'entretien.

En particulier, on peut utiliser le schéma thérapeutique suivant qui comporte l'administration du chlorhydrate d'amiodarone comme principe actif d'abord en dose de charge ensuite en dose d'entretien à savoir :

- **Traitement d'attaque** : 800mg / jour ou 4 comprimés dosés chacun à 200mg de chlorhydrate d'amiodarone pendant 2 semaines, le traitement débutant précocement après la phase aiguë de l'infarctus du myocarde par exemple dès le 5ème jour.

- **Traitement d'entretien** : 400mg / jour ou 2 comprimés dosés chacun à 200mg de chlorhydrate d'amiodarone pendant 3 à 4 mois puis 200mg de chlorhydrate d'amiodarone pendant au moins 12 mois.

Selon un autre aspect de l'invention, l'administration du dérivé de benzofuranne à activité antiarythmique ou d'un de ses sels pharmaceutiquement acceptables peut être réalisée en association étroite c'est-à-dire simultanée ou séquentielle avec l'administration d'au moins un agent cardioactif supplémentaire de manière à renforcer l'effet prophylactique recherché et/ou traiter la maladie cardiaque sous-jacente.

Par conséquent, un autre objet de l'invention se rapporte à l'utilisation d'un dérivé de benzofuranne à activité antiarythmique, préférentiellement l'amiodarone ou la dronédarone, ou d'un sel pharmaceutiquement acceptable de ce dérivé pour la préparation de compositions pharmaceutiques à administrer en association simultanée ou séquentielle, à au moins un agent cardioactif supplémentaire, pour la réduction de la mortalité d'origine cardiaque chez des patients présentant une fonction ventriculaire gauche réduite après infarctus du myocarde, sans troubles du rythme nécessitant un traitement antiarythmique.

Selon cette variante, on peut associer le principe actif, par exemple l'amiodarone, la dronédarone ou un de leurs sels pharmaceutiquement acceptables, et au moins un agent cardioactif supplémentaire choisi parmi un diurétique, un inhibiteur de l'enzyme de conversion de l'angiotensine, un inhibiteur de l'angiotensine II, un inhibiteur calcique, un agent cardiotonique, un agent β-bloquant, un dérivé nitré et un inhibiteur de la vitamine K.

A titre d'exemples non limitatifs, on citera par la suite des agents cardioactifs qui peuvent être associés au principe actif en question, ces agents étant dénommés par leur Dénomination Commune Internatinale (DCI).

Ainsi, on peut sélectionner:

- le diurétique parmi le furosémide, l'hydrochlorothiazide, la métolazone, l'amiloride et la spironolactone, ces composés étant administrés à des doses thérapeutiquement actives choisies entre 1 et 200mg/jour.

- l'inhibiteur de l'enzyme de conversion de l'angiotensine parmi le captopril, l'énalapril, le fosinopril, le quinapril, le ramipril, le lisinopril, le cilazapril et le périndopril, ces composés étant administrés à des doses thérapeutiquement actives choisies entre 2 et 150mg/jour.

- l'inhibiteur de l'angiotensine II parmi l'irbésartan, le losartan, le candesartan, le valsartan, le zolasartan, le telminsartan, l'éprosartan, ces composés étant administrés à des doses thérapeutiquement actives choisies entre 1 et 200mg / jour. L'irbésartan représente toutefois un inhibiteur de l'angiotensine II préféré.

- l'inhibiteur calcique parmi la nifédipine, la nicardipine, la lacidipine, la félodipine, l'amlodipine, le diltiazem et le vérapamil, ces composés étant administrés à des doses thérapeutiquement actives choisies entre 1 et 500 mg / jour.

- l'agent cardiotonique parmi l'acétyldigitoxine, la digitoxine et la digoxine, ces composés étant administrés à des doses thérapeutiquement actives choisies entre 0,1 et 2mg/jour.

- l'agent β-bloquant parmi le timolol, l'aténolol, le pindolol, le bisoprolol, l'acébutolol, le propranolol, le métoprolol, le nadolol, le tertatolol, l'alprénolol, le betaxolol, le céliprolol et l'oxprénolol, ces composés étant administrés à des doses thérapeutiquement actives choisies entre 10 et 500 mg/jour.

- le dérivé nitré parmi le nicorandil, le mononitrate d'isosorbide, le dinitrate d'isosorbide et la trinitrine, ces composés étant administrés à des doses thérapeutiquement actives choisies entre 2 et 200mg/jour.

- l'antagoniste de la vitamine K parmi la warfarine, l'acénocoumarole et la phenprocoumone, ces composés étant administrés à des doses thérapeutiquement actives choisies entre 1 et 50mg/jour.

Les associations avec un inhibiteur de l'enzyme de conversion de l'angiotensine ou avec un inhibiteur de l'angiotensine II représentent toutefois des associations préférées.

Cependant, l'amiodarone ou ses sels pharmaceutiquement acceptables, ne représente pas le dérivé de benzofuranne à activité antiarythmique de choix pour la constitution par exemple d'une association simultanée représentée par une unité d'administration unique étant donné sa durée de vie particulièrement prolongée par rapport aux autres agents cardioactifs qui lui seraient associés.

L'efficacité prophylactique des dérivés de benzofuranne à activité antiarythmique et de leurs sels pharmaceutiquement acceptables vis-à-vis de la mortalité post infarctus a été mise en évidence par l'intermédiaire du chlorhydrate d'amiodarone, lors d'une étude clinique prospective, multicentrique et en double aveugle (avec une approche en triple aveugle) avec randomisation (répartition au hasard) des patients en 2 groupes définis à partir de la fraction d'éjection ventriculaire gauche.

Cette étude, qui s'est étalée sur une période de plusieurs années, a été pratiquée chez des patients à haut risque présentant une fonction ventriculaire gauche déficiente après infarctus du myocarde récent. Elle a consisté à démontrer sur une période de 2 ans, une diminution de la mortalité cardiaque, de la mortalité cardiaque d'origine arythmique cumulée ou non aux événements arythmiques non fatals causant un arrêt cardiaque et enfin une diminution de la mortalité subite d'origine arythmique (mort subite).

## ETUDE CLINIQUE

### I. Sélection des patients

La randomisation des patients a été effectuée entre les 5ème et 21ème jours après infarctus du myocarde. Durant cette période, on a vérifié les critères d'inclusion et d'exclusion suivants pour chaque patient des deux sexes susceptible d'être enrôlés dans l'essai à savoir :

*Critères d'inclusion*

1) présence d'un infarctus du myocarde

2) fraction d'éjection inférieure ou égale à 40% par ventriculographie radionuclide standardisée

3) âge supérieur à 18 ans et inférieur à 75 ans

Un enregistrement Holter a également été pratiqué durant 24 heures mais les résultats n'ont pas été retenus pour l'inclusion.

*Critères d'exclusion*

1) contre-indications à l'amiodarone

2) statut particulier du patient notamment femmes en age de procréation, maladie non cardiaque ou préexistante susceptible d'abréger de manière significative une survie de 2 ans ou toute condition requérant un traitement avec des antidépresseurs tricycliques, la phénytoïne, la lidoflazine, la prenylamine ou la vincamine

3) nécessité d'une thérapie antiarythmique autre que des β-bloquants ou la digitale au moment de l'incorporation dans l'essai

4) angor sévère ne répondant pas à un traitement conventionnel

5) insuffisance cardiaque grave ne répondant pas à un traitement conventionnel

6) nécessité d'une chirurgie cardiaque

Chacun des 1486 patients sélectionnés selon ces critères ont été inclus dans l'étude et randomisés dans deux groupes entre 5 et 21 jours après l'infarctus du myocarde pour recevoir soit le chlorhydrate d'amiodarone soit le placebo. Ces groupes ont été constitués sur base de la fraction d'éjection c'est-à-dire :

- un groupe de 797 patients dotés d'une fraction d'éjection supérieure à 30% et égale ou inférieure à 40% ci-après "Groupe I"
- un groupe de 689 patients dotés d'une fraction d'éjection inférieure à 30% ci-après "Groupe II"

## II. Durée et traitement

L'essai a duré pendant 4 années, la période de recrutement s'étalant sur 3 ans de sorte que les patients recrutés durant les 2 premières années ont été traités pendant 2 ans et ceux recrutés dans la dernière année ont été traités jusqu'à la fin de l'essai. Le traitement minimum a été, par conséquent, de 1 an.

Ce traitement a été instauré en double aveugle à partir de comprimés contenant soit le placebo soit 200mg de chlorhydrate d'amiodarone, selon le schéma thérapeutique suivant:

a) *Dose de charge*:
   800mg soit 4 comprimés/jour en deux prises pendant 2 semaines.

b) *Dose d'entretien*:

* 400mg soit 2 comprimés / jour en deux prises à partir de la 3éme semaine pendant 3 mois et 2 semaines.
* 200mg soit 1 comprimé / jour durant 20 mois.

Une co-médication a également été instaurée à savoir odes inhibiteurs de l'enzyme de conversion de l'angiotensine chez 77% des patients, des β-bloquants chez 44%, des antagonistes de la vitamine K chez 29%, de la digoxine chez 23%, des antagonistes du calcium chez 14%.

Des enregistrements Holter pratiqués au cours de l'essai ont révélé qu'environ 40% des patients présentaient plus de 10 extrasystoles ventriculaires par heure ou de 3 extrasystoles bi ou tri-géminées

## III. RESULTATS

Les différences de survie enregistrées ont été analysées par la méthode de Kaplan-Meyer (p significatif si $\leq 0,05$). En prenant en compte :

- la mortalité totale dans l'essai, c'est-à-dire la mortalité due à toute cause qu'elle soit cardiaque, non cardiaque ou inconnue
- ou la mortalité cardiaque totale, c'est-à-dire la mortalité due à des causes cardiaques,

on n'a pas enregistré, sur une période de 24 mois, une différence significative entre le taux de survie dans le groupe traité et le taux de survie dans le groupe placebo. Toutefois, on n'a relevé aucun effet délétère provoqué par le chlorhydrate d'amiodarone sur la période considérée. En outre, on n'a pas observé de réduction significative de la mortalité totale dans les Groupes I et II sur la même période de 24 mois. Cependant, on a pu mettre en évidence un effet préventif précoce de la mortalité totale dans le Groupe I sur la période des 6 premiers mois (p = 0,06) et notamment sur la période des 3 premiers mois (p < 0,05).

Par ailleurs, on n'a observé, par rapport au groupe placebo aucun effet significatif de réduction de la mortalité totale sur une période de 24 mois chez des patients avec histoire d'infarctus.

Cependant une tendance à prevenir la mortalité totale chez des patients sans histoire d'infarctus (avec infarctus inaugural ) a pu être relevée sur la même période de 24 mois (p < 0,05 sur 3 mois, p = 0,06 sur 6 mois et p = 0,07 sur 12 mois).

D'autres résultats obtenus lors de cet essai randomisé sont répertoriés ci-après en référence aux dessins ci-annexés sur lesquels :

a) les abréviations utilisées possèdent les significations suivantes :

" PL " signifie " placebo "
" Am " et "Amiodarone" signifie " chlorhydrate d'amiodarone "
" St. 1 " signifie " Groupe I "

" St. 2 " signifie " Groupe II "

" MI " signifie "infarctus du myocarde non inaugural "

" noMi " signifie "infarctus du myocarde inaugural"

" * " signifie p < 0,05

" ** " signifie p < 0,01

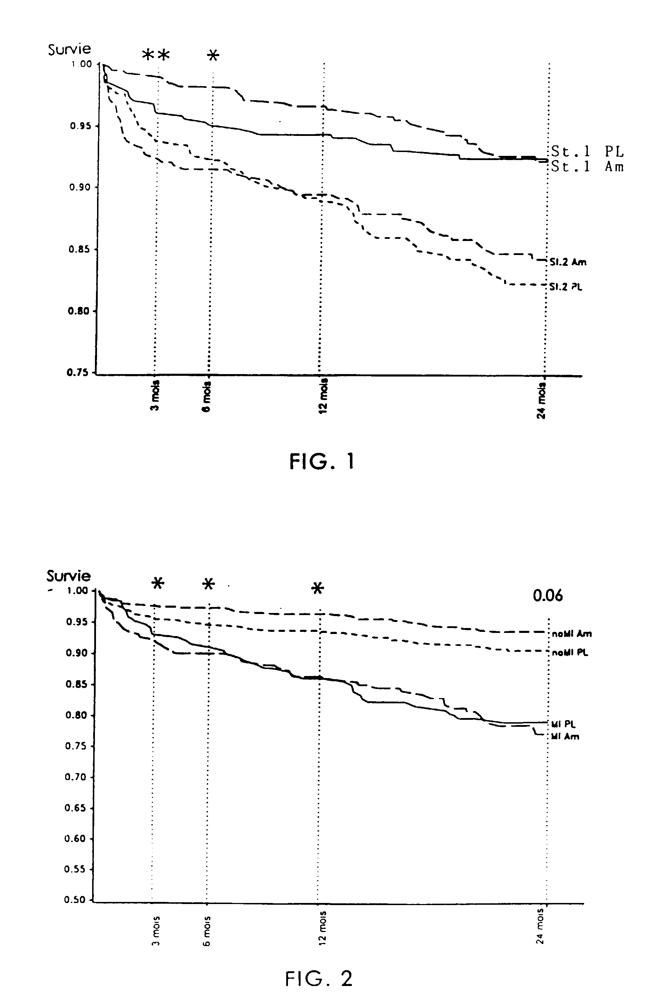

b) la Figure 1 représente le taux de patients dans les Groupes I et II survivant durant 24 mois à la mort d'origine cardiaque.

Ces résultats montrent que le chlorhydrate d'amiodarone ne diminue pas de manière significative, par rapport au groupe placebo, le taux de mortalité d'origine cardiaque sur une période de 24 mois dans les 2 groupes en question. Toutefois, un effet prophylactique précoce a pu être mis en évidence durant les 6 premiers mois dans le Groupe I (p < 0,05 sur 6 mois) et plus particulièrement dans les 3 premiers mois (p <0,01 sur 3 mois).

c) la Figure 2 représente le taux de patients survivant durant 24 mois à la mort cardiaque après infarctus inaugural.

On n'observe, suite à cet essai et par rapport au groupe placebo, aucun effet significatif, de réduction de la mortalité d'origine cardiaque sur une période de 24 mois chez des patients avec histoire d'infarctus. Par contre, une tendance à prévenir la mortalité d'origine cardiaque chez des patients sans histoire d'infarctus a pu être décelée sur la même période de 24 mois (p = 0,06 sur 24 mois) tandis qu'une diminution significative de ce type de mortalité a été relevée sur une période de 3 mois, 6 mois et 12 mois (p < 0,05 sur 3,6 et 12 mois).

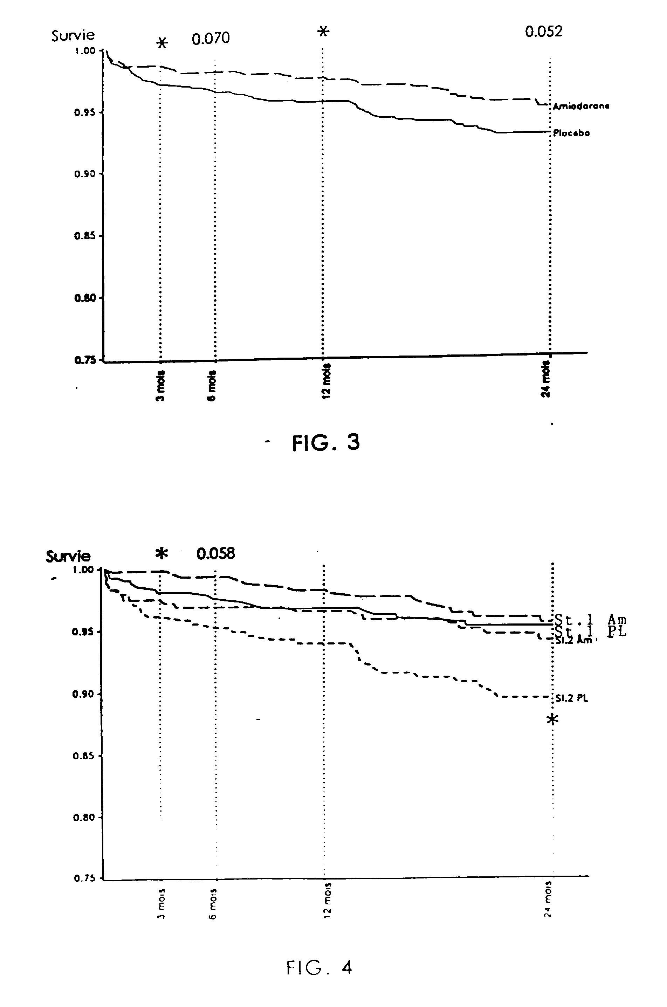

d) la Figure 3 représente le taux de patients survivant à la mort cardiaque d'origine arythmique sur une période de 24 mois.

Les résultats obtenus montrent une réduction significative de ce type de mortalité, par rapport au groupe placebo, sur une période de 3 mois (p < 0,05), 6 mois (p < 0,05) et 24 mois (p = 0,052).

Par exemple, on a obtenu 44% de réduction de la mortalité cardiaque d'origine arythmique à 6 mois et 30% à 24 mois.

e) la Figure 4 représente le taux de patients dans les Groupes I et II survivant sur une période de 24 mois à la mort cardiaque d'origine arythmique.

On peut déduire de ces résultats une réduction significative de ce type de mortalité par rapport au groupe placebo sur une période de 3 mois (p < 0,05) et 6 mois (p = 0,058) dans le Groupe I et sur une période de 24 mois (p < 0,05) dans le Groupe II.

Ainsi, on a observé un effet prophylactique significatif encore plus marqué dans le Groupe II que dans le Groupe I c'est-à-dire chez des patients avec une fraction d'éjection ventriculaire gauche particulièrement réduite (< 30%).

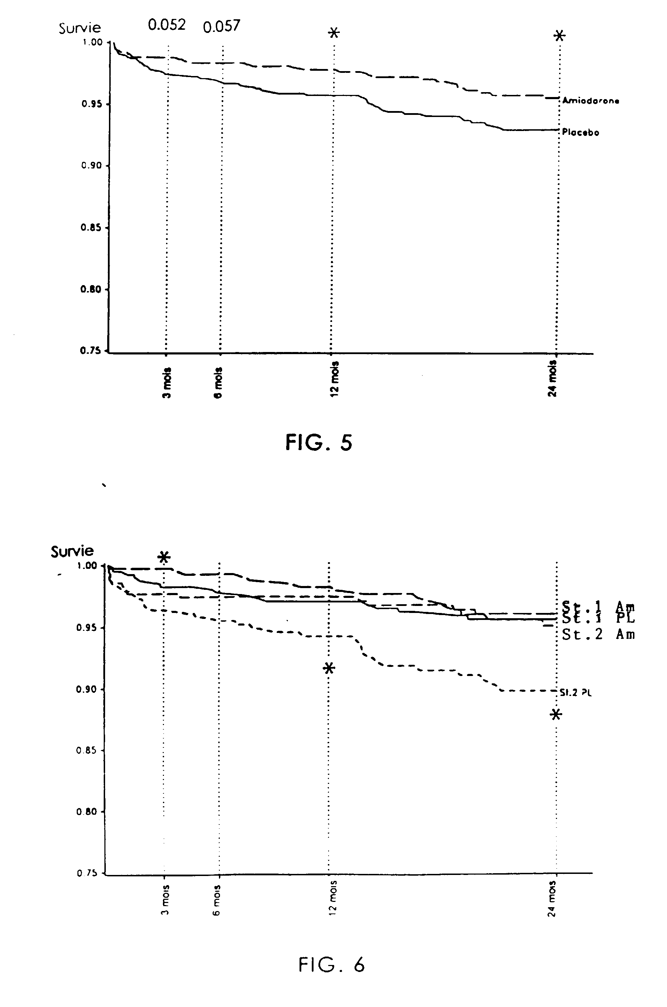

f) la Figure 5 représente le taux de patients survivant à la mort subite d'origine arythmique sur une période de 24 mois.

Les résultats obtenus mettent en lumière une réduction significative de ce type de mortalité par rapport au groupe placebo (p = 0.052 sur 3 mois. 0.057 sur 6 mois, < 0,05 sur 12 mois et < 0,05 sur 24 mois).

En outre, on a enregistré les % suivants de réduction de ce type de mortalité :

53% de réduction à 3 mois

48% de réduction à 6 mois

48% de réduction à 12 mois

37% de réduction à 24 mois

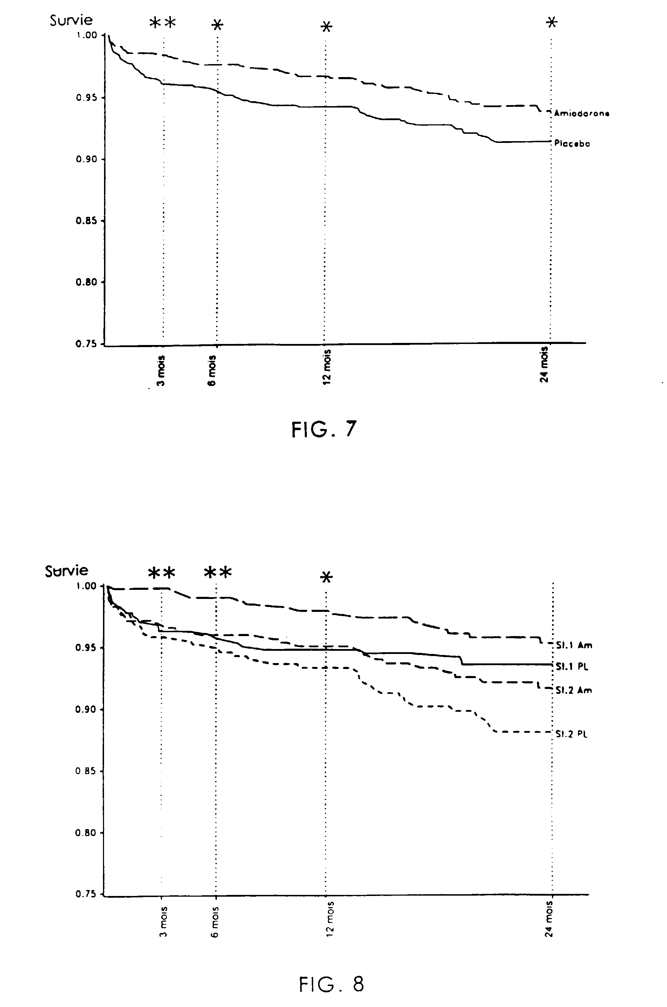

g) la Figure 6 représente le taux de patients dans les Groupes I et II survivant, sur une période de 24 mois, à la mort subite d'origine arythmique.

Par rapport au placebo, une réduction significative de ce type de mortalité a été enregistrée, sur la période considérée, dans les deux groupes ayant reçu le chlorhydrate d'amiodarone. Toutefois un effet réducteur encore plus marqué a été relevé dans le Groupe II (p < 0,05 sur 12 mois et sur 24 mois) c'est-à-dire dans le groupe de patients présentant une fraction d'éjection fortement amoindrie (< 30%).

h) la Figure 7 représente le taux cumulé de patients survivant sur une période de 24 mois, soit à la mort cardiaque d'origine arythmique soit aux arrêts cardiaques non fatals.

Ces résultats mettent en évidence une diminution significative sur une période de 24 mois, de ce type d'événement par rapport au groupe placebo (p < 0,01 sur 3 mois et p < 0,05 sur 6 mois, 12 mois et 24 mois). En outre, les % de diminution suivants ont été enregistrés :

59% de diminution à 3 mois
45% de diminution à 6 mois
41% de diminution à 12 mois
28% de diminution à 24 mois

I) la Figure 8 représente le taux cumulé de patients dans les Groupes I et II survivant sur une période de 24 mois soit à la mort cardiaque d'origine arythmique soit aux arrêts cardiaques non fatals.

Dans le Groupe II une tendance à prévenir ce type d'événement a été relevée sur une période de 24 mois alors que dans le Groupe I, une réduction significative de ce type d'événement a été obtenue avec le chlorhydrate d'amiodarone par rapport au groupe placebo sur une période d'au moins 12 mois (p <0,01 sur 3 et 6 mois et p < 0,05 sur 12 mois).

## Revendications

1. Utilisation de dérivés de benzofuranne à activité antiarythmique ou d'un de leurs sels pharmaceutiquement acceptables comme principes actifs pour la préparation de compositions pharmaceutiques destinées à réduire la mortalité d'origine cardiaque chez des patients présentant une fonction ventriculaire gauche réduite après infarctus du myocarde, sans troubles du rythme nécessitant un traitement antiarythmique.

2. Utilisation selon la Revendication 1 caractérisée en ce que le dérivé de benzofuranne est l'amiodarone ou un de ses sels pharmaceutiquement acceptables.

3. Utilisation selon la Revendication 1 caractérisée en ce que le dérivé de benzofuranne est la dronédarone ou un de ses sels pharmaceutiquement acceptables.

4. Utilisation selon la Revendication 1 caractérisée en ce que le dérivé de benzofuranne est la deséthylamiodarone ou un de ses sels pharmaceutiquement acceptables.

5. Utilisation selon la Revendication 1 caractérisée en ce que le dérivé de benzofuranne est la N-desbutyldronédarone.

6. Utilisation selon une des Revendications 1 à 5 caractérisée en ce que le sel pharmaceutiquement acceptable est le chlorhydrate.

7. Utilisation du chlorhydrate d'amiodarone comme principe actif pour la préparation de compositions pharmaceutiques destinées à réduire la mortalité d'origine cardiaque chez des patients présentant une fonction ventriculaire gauche réduite après infarctus du myocarde, sans troubles du rythme nécessitant un traitement antiarythmique.

8. Utilisation selon une des Revendications 1 à 7 caractérisée en ce que les compositions pharmaceutiques sont destinées à réduire la mortalité cardiaque d'origine arythmique.

9. Utilisation selon une des Revendications 1 à 7 caractérisée en ce que les compositions pharmaceutiques sont destinées à réduire la mortalité cardiaque d'origine arythmique comprenant les arrêts cardiaques non fatals.

10. Utilisation selon une des Revendications 1 à 7 caractérisée en ce que les compositions pharmaceutiques sont destinées à réduire la mortalité subite d'origine arythmique ou mort subite.

11. Utilisation selon une des Revendications 1 à 7 caractérisée en ce que les compositions pharmaceutiques sont destinées à réduire la mortalité d'origine cardiaque chez des patients présentant une fonction ventriculaire gauche réduite se reflétant par une fraction d'éjection $\leq$ 40%.

12. Utilisation selon la Revendication 11 caractérisée en ce que la fraction d'éjection est supérieure à 30% et égale ou inférieure à 40%.

13. Utilisation selon la Revendication 11 caractérisée en ce que la fraction d'éjection est inférieure à 30%.

14. Utilisation selon une des Revendications 1 à 13 caractérisée en ce que l'on prépare des compositions pharmaceutiques pour administration par voie orale, sublinguale, nasale, inhalée, parentérale, topique, transdermique ou rectale.

15. Utilisation selon la Revendication 14 caractérisée en ce que l'on prépare des compositions pharmaceutiques pour administration par voie orale.

16. Utilisation selon la Revendication 14 caractérisée en ce que l'on prépare des compositions pharmaceutiques pour administration par voie parentérale.

17. Utilisation selon la Revendication 14 ou 15 caractérisée en ce que l'on prépare des compositions pharmaceutiques contenant de 50 à 600mg de principe actif pour administration par voie orale.

18. Utilisation selon la Revendication 14 ou 16 caractérisée en ce que l'on prépare des compositions pharmaceutiques contenant de 10 à 250mg de principe actif pour administration par voie parentérale.

19. Utilisation selon la Revendication 15 ou 17 caractérisée en ce que l'on prépare des compositions pharmaceutiques pour l'administration, par voie orale, de doses journalières de 100 à 800mg d'amiodarone ou d'un de ses sels pharmaceutiquement acceptables.

20. Utilisation selon la Revendication 19 caractérisée en ce que l'on prépare des compositions pharmaceutiques pour l'administration, par voie orale, de doses journalières de charge de 400 à 800mg d'amiodarone ou d'un de ses sels pharmaceutiquement acceptables.

21. Utilisation selon la Revendication 19 caractérisée en ce que l'on prépare des compositions pharmaceutiques pour l'administration, par voie orale, de doses journalières d'entretien de 200 à 300mg d'amiodarone ou d'un de ses sels pharmaceutiquement acceptables.

22. Utilisation selon la Revendication 21 caractérisée en ce que la dose journalière d'entretien est de 200mg d'amiodarone ou d'un de ses sels pharmaceutiquement acceptables.

23. Utilisation selon une des Revendications 1 à 22 caractérisée en ce que les compositions pharmaceutiques sont administrées en association simultanée ou séquentielle à au moins un agent cardioactif supplémentaire.

24. Utilisation selon la Revendication 23 caractérisée en ce que l'agent cardioactif supplémentaire est choisi parmi un diurétique, un inhibiteur de l'enzyme de conversion de l'angiotensine, un inhibiteur de l'angiotensine II, un inhibiteur calcique, un agent cardiotonique, un agent β-bloquant, un dérivé nitré et un inhibiteur de la vitamine K.

25. Utilisation selon la Revendication 24 caractérisée en ce que :

- le diurétique est choisi parmi le furosémide, l'hydrochlorothiazide, la métolazone, l'amiloride et la spironolactone
- l'inhibiteur de l'enzyme de conversion de l'angiotensine est choisi parmi le captopril, l'énalapril, le fosinopril, le quinapril, le ramipril, le lisinopril, le cilazapril et le périndopril
- l'inhibiteur de l'angiotensine II est choisi parmi l'irbésartan, le losartan, le candesartan, le valsartan, le zolasartan, le telminsartan et l'éprosartan
- l'inhibiteur calcique est choisi parmi la nifédipine, la nicardipine, la lacidipine, la félodipine, l'amlodipine, le diltiazem et le vérapamil
- l'agent cardiotonique est choisi parmi l'acétyldigitoxine, la digitoxine et la digoxine
- l'agent β-bloquant est choisi parmi le timolol, l'aténolol, le pindolol, le bisoprolol, l'acébutolol, le propranolol, le métoprolol, le nadolol, le tertatolol, l'alprénolol, le betaxolol, le céliprolol et l'oxprénolol
- le dérivé nitré est choisi parmi le nicorandil, le mononitrate d'isosorbide, le dinitrate d'isosorbide et la trinitrine
- l'antagoniste de la vitamine K est choisi parmi la warfarine, l'acénocoumarole et la phenprocoumone.

26. Utilisation selon une des Revendications 23 à 25 caractérisée en ce que les compositions pharmaceutiques sont administrées en association simultanée ou séquentielle à un inhibiteur de l'enzyme de conversion de l'angiotensine

**EP 0 796 617 A1**

ou à un inhibiteur de l'angiotensine II.

27. Utilisation selon la Revendication 26 caractérisée en ce que les compositions pharmaceutiques sont administrées en association simultanée ou séquentielle à l'irbésartan.

28. Utilisation selon une des Revendications 23 à 27 caractérisée en ce que les compositions pharmaceutiques contiennent l'amiodarone ou un de ses sels pharmaceutiquement acceptables.

29. Utilisation selon une des Revendications 23 à 27 caractérisée en ce que les compositions pharmaceutiques contiennent la dronédarone ou un de ses sels pharmaceutiquement acceptables.

30. Utilisation selon la Revendication 28 ou 29 caractérisée en ce que le sel pharmaceutiquement acceptable est le chlorhydrate.

FIG. 1

FIG. 2

FIG. 3

FIG. 4

FIG. 5

FIG. 6

17

FIG. 7

FIG. 8

# EP 0 796 617 A1

**Office européen des brevets**

## RAPPORT DE RECHERCHE EUROPEENNE

Numero de la demande

EP 97 40 0593

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,D | AM. J. CARD.,<br>vol. 72, no. 16, 1993,<br>pages 95f-98f, XP000607602<br>CAMM A.F. ET AL.: "the european<br>myocardial infarct amiodarone trial<br>(EMIAT)" | 1,2,8-14 | A61K31/34 |
| Y | * le document en entier * | 3-7,<br>15-30 | |
| | --- | | |
| X,D | ARCH. MAL. COEUR,<br>vol. 94, no. II, 1991,<br>pages 67-69, XP000607681<br>MUNOZ A.: "amiodarone et prévention<br>secondaire, l'essai EMIAT" | 1,2,8-14 | |
| Y | * le document en entier * | 3-5,<br>15-30 | |
| | --- | | |
| X | AM. J. CARDIOL.,<br>vol. 72, no. 16, 1993,<br>pages 70f-74f, XP000607627<br>GREENE H.L.: "the CASCADE study:<br>randomized antiarrhythmic drug therapy in<br>survivors of cardiac arrest in Seattle" | 1,2,8,9,<br>11,12 | |
| Y | * page 73F, colonne de gauche * | 3-7,10,<br>13-30 | |
| | --- | | |
| X | ANN. INTER. MED.,<br>vol. 122, no. 9, 1995,<br>pages 689-700, XP000607576<br>PODRID P.J.: "amiodarone: reevaluation of<br>an old drug" | 1,2 | |
| Y | * page 693-694 * | 3-30 | |
| | --- | | |
| | -/-- | | |

**DOMAINES TECHNIQUES RECHERCHES (Int.Cl.6)**

A61K

Le présent rapport a été établi pour toutes les revendications

| Lieu de la recherche | Date d'achèvement de la recherche | Examinateur |
|---|---|---|
| LA HAYE | 21 Mai 1997 | Trifilieff-Riolo, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
....................
& : membre de la même famille, document correspondant

EPO FORM 1503 03.82 (P04C02)

19

Office européen
des brevets

**RAPPORT DE RECHERCHE EUROPEENNE**

Numero de la demande

EP 97 40 0593

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|-----------|----------------------------------------------------------------------------------|-------------------------|-------------------------------------|
| X,D | J. AM. COLL. CARDIOL., vol. 16, no. 7, 1990, pages 1711-1718, XP000607559 BURKART ET AL.: "effect of antiarrhythmic therapy on mortality in survivors of myocardial infarction (BASIS)" | 1,2 | |
| Y | * le document en entier * --- | 3-30 | |
| X,D | FR 2 626 176 A (SOORIANARAIN BALIGADOO) 28 Juillet 1989 | 1,2, 8-10, 14-25,28 | |
| Y | * le document en entier * --- | 3-7, 11-13, 26,27, 29,30 | |
| Y,D | FR 2 550 091 A (SANOFI) 8 Février 1985 * page 2, ligne 5 * --- | 1,4 | |
| Y,D | US 5 223 510 A (GUBIN ET AL.) 29 Juin 1993 colonnes 21 et 22, deux premiers composés ----- | 1,3,5 | DOMAINES TECHNIQUES RECHERCHES (Int.Cl.6) |

Le présent rapport a été établi pour toutes les revendications

| Lieu de la recherche | Date d'achèvement de la recherche | Examinateur |
|----------------------|-----------------------------------|-------------|
| LA HAYE | 21 Mai 1997 | Trifilieff-Riolo, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................

& : membre de la même famille, document correspondant

EPO FORM 1503 03.82 (P04C02)